# EUROPEAN PATENT APPLICATION

(11) **EP 1 249 439 A1**
(43) Date of publication of application: **16.10.2002**
(21) Application number: 01201371.0
(22) Date of filing: 13.04.2001
(51) Int. Cl.: C07B 41/06, C07C 67/347, C07C 67/54, C07C 69/716, C07C 45/50

(54) **Continuous hydroformylation process**

(71) Applicant: DSM N.V., 6411 TE Heerlen (NL)
(72) Inventor: Sielcken, Otto Erik, 6136 BG Sittard (NL); Smits, Hubertus Adrianus, 6212 GC Maastricht (NL); Toth, Imre, 6166 GM Geleen (NL)

(57) **Abstract**

A continuous hydroformylation process for forming an aldehyde comprising:
A) reacting an olefinically unsaturated compound containing from 6 to 30 carbon atoms, carbon monoxide, hydrogen, and a rhodium-bisphosphite ligand complex catalyst to form a reaction mixture
B) evaporating at least a portion of said liquid reaction mixture to isolate aldehyde product
C) recycling the catalyst after the evaporation,
wherein said evaporation is accomplished in a vacuum evaporator with a short mean residence time and a moderate thermal exposure of the liquid reaction mixture in the evaporator.

## Description

The invention relates to a continuous hydroformylation process for forming an aldehyde comprising:
A) reacting an olefinically unsaturated compound containing from 6 to 30 carbon atoms, carbon monoxide, hydrogen, and a rhodium-bisphosphite ligand complex catalyst to form a reaction mixture
B) evaporating at least a portion of said liquid reaction mixture to isolate aldehyde product
C) recycling the catalyst after the evaporation.

It is well known in the art that organophosphites may be employed as catalyst ligands for rhodium based hydroformylation catalysts and that such catalysts exhibit exceptional activity and regioselectivity for producing aldehydes via olefin hydroformylation. For instance, U.S. Pat. Nos. 4,668,651 and 4,769,498 fully detail such hydroformylation.

However, despite the benefits attendant with such rhodium-organophosphite complex catalyzed hydroformylation processes, stability of the ligand and catalyst remains a primary concern. Although hydroformylation processes are performed at moderate temperatures (<110°C), heating rhodium organophosphite catalysts above these temperatures has led to decomposition of the catalyst and ligand and has generally resulted in irreversible ligand and/or rhodium metal loss. For example U.S. Pat. Nos 5,288,918 and 5,763,670 describe the decomposition of rhodium bisorganophosphite complexes and ligands which occurs as the result of elevated temperatures. As a result of this decomposition, the activity and efficiency of the hydroformylation process are lowered and the cost of operating a hydroformylation facility is greatly increased.

The thermal decomposition of rhodium bisorganophosphite complexes is most apparent during the evaporation phase of the hydroformylation process. When producing aldehydes with high boiling points (aldehydes with at least 6 carbon atoms), high temperatures are required to vaporize these compounds so they may be separated and isolated from other materials which are present in the reaction mixture. These harsh conditions inevitably result in rhodium complex catalyst decomposition during product isolation. Although techniques aimed at aldehyde separation at lower temperatures, such as reduced pressure evaporation, have met with limited success, a hydroformylation process which allows for aldehyde collection while exposing the catalyst to only very brief periods of elevated temperature would provide for increased catalyst life and thus a more efficient hydroformylation process, especially in light of the high cost of rhodium metal and of the preferred bisorganophosphite ligands.

The object of the invention is an improved process for separating rhodium complex catalyst and aldehyde product(s) in a rhodium-organophosphite complex catalyzed hydroformylation process.

This object is achieved in that said evaporation is accomplished in a vacuum evaporator with a short mean residence time and a moderate thermal exposure of the liquid reaction mixture in the evaporator.

It has been found that in the process of the present invention rhodium complex catalyst decomposition during product isolation is avoided or at least minimized.

Examples of suitable evaporators are a (wiped) falling film evaporator or a short path distillation apparatus.

In a preferred embodiment of the hydroformylation process of the present invention, the evaporation is performed at a pressure below 200 mbar, more preferably below 50 mbar, more preferably below 20 mbar, and still more preferably the pressure is below 10 mbar.

The mean residence time of the liquid reaction mixture in the evaporation is preferably less than 15 minutes, more preferably less than 10 minutes and more preferably less than 5 minutes.

In a preferred embodiment of the present invention the rhodium-bisphosphite complex catalyst comprises a bisphosphite ligand of a formula selected from the group consisting of: 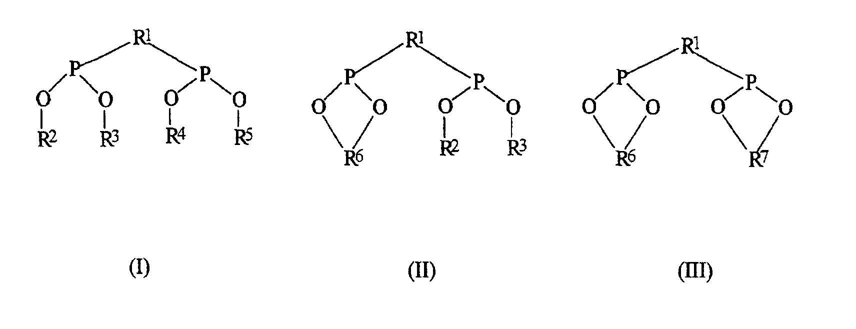 wherein each R¹ represents a divalent radical selected from a group consisting of alkylene, alkylene-(Q)ₙ-alkylene, arylene and arylene-(Q)ₙ-arylene, and wherein each alkylene radical individually contains from 2 to 18 carbon atoms and is the same or different, and wherein each arylene radical individually contains from 6 to 18 carbon atoms and is the same or different; wherein each Q individually represents a divalent bridging group of ―O― or―CR'R"― wherein each R' and R" radical individually represents hydrogen or a methyl radical; and wherein each n individually has a value of 0 or 1,
wherein R², R³, R⁴, and R⁵ might be the same or different and each is individually represented by the structure of (VI) or (VII), 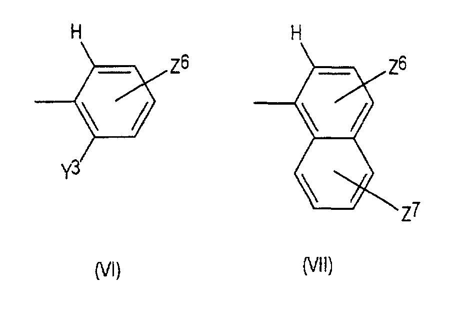 wherein R⁶ and R⁷ might be the same or different and each is individually represented by the structure of (VIII) or (IX), 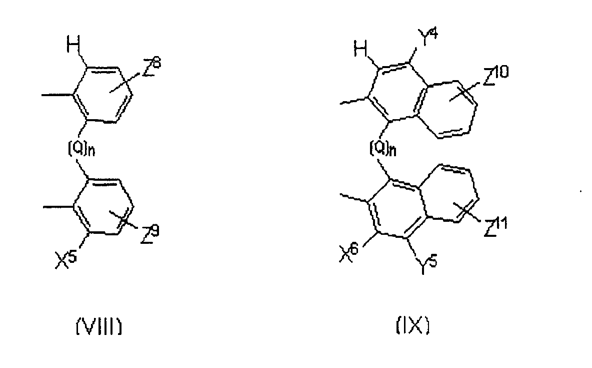 wherein X¹, X², X³, X⁴, X⁵ and X⁶ might be the same or different and each individually represents an organic radical, wherein Y¹, Y², Y³, Y⁴ and Y⁵ are the same or different and each represents a hydrogen or alkyl radical, wherein Z¹, Z², Z³, Z⁴, Z⁵, Z⁶, Z⁷, Z⁸, Z⁹, Z¹⁰ and Z¹¹ might be the same or different and each represent a hydrogen or an organic radical placed at any remaining position of the aryl rings of structures (IV) to (V),

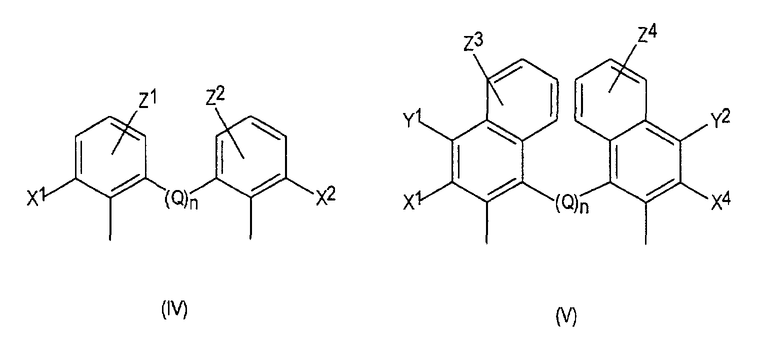

In a more preferred embodiment of the present invention R¹ is represented by the structure of (IV),(V), (VII), (VIIIO, (IX), wherein (Q)ₙ is the same as above, wherein X¹ is the same as X² and Z¹ is the same as Z² in Formula (IV), X³ is the same as X⁴, Z³ is the same as Z⁴, and Y¹ and Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).

Still more preferably said hydroformylation process is performed wherein said ligand used is chosen from the group consisting of [3,3'-bis(t-butyl)-5,5'-dimethoxy-1,1'-biphenyl-2,2'-diyl]-bis(oxy)]-bis(dibenzo[d,f] [1,3,2])dioxaphosphepin, 3,3'-bis(carboxyisopropyl)-1,1'-binaphthyl-2,2-diyl-bis[bis(1-naphthyl)]phosphite and 3,3'-bis(carboxymethyl)-1,1'-binaphthyl-2,2'-diyl-bis[bis(2,5-di-t-butyl)]phosphite.

Accordingly, the subject invention encompasses preventing or minimizing the catalyst deactivation of rhodium-bisphosphite complex catalyzed hydroformylation processes for producing aldehydes, by carrying out the evaporation step of said process with a vacuum evaporator with a short mean residence time and moderate thermal exposure of the catalyst such as a (wiped) falling film evaporator or a short path distillation apparatus. Such vacuum evaporators consist of a heating element which heats a portion of the reaction mixture as a thin liquid film and a condensation surface which collects the aldehyde containing product mixture. For the purposes of this invention, the term reaction mixture is meant to mean the compounds contained in the solution or suspension where the chemical reaction known in the art as hydroformylation is performed or occurs. The evaporation functionality and condensation functionality may be physically separated into separate housings as in a standard (wiped) falling evaporator with an external condenser or it may be integrated into a single housing as in a short path distillation apparatus.

Preferably the heating surface of the evaporator of the present invention is maintained at a temperature below 125°C, and more preferably this surface is maintained below 110°C. The cooling surface of the evaporator may be cooled with any convenient mode known to those skilled in the art. More preferably, the cooling surface is cooled by water, air, a brine solution, evaporating ammonia, or by evaporating propane, propene, butane or butenes.

It is also preferable to perform the evaporation of the present invention by distilling a thin film of the reaction mixture to separate the product aldehyde, and such a technique is a preferred embodiment of the present invention. Preferably the reaction mixture film of the present invention has a thickness between 0.05 - 5 mm. Still more preferably, the film thickness is between 0.05 and 0.5 mm. It is also advantageous to mechanically wipe the evaporating surface, thereby obtaining improved heat and mass transfer and preventing thermal and concentration gradients from being formed along the axis of the liquid film thickness. Wiping also makes the residence time distribution more narrow which is beneficial to the catalyst stability.

As stated above, the subject invention resides in the discovery that decomposition of such rhodium-bisphosphite complex catalysts can be minimized or prevented by performing the product/catalyst separation step of the hydroformylation process in a vacuum evaporation apparatus. This beneficial effect results from several factors. First, the time the distillation mixture spends in contact with the heating element is reduced in the vacuum evaporator, which in turn minimizes the time in which the catalyst is exposed to such a threatening high temperature process. The short contact time obtained by the present invention results from the placement of a thin film of the reaction mixture over the heating element, which allows for rapid heating of the evaporation mixture so that the product aldehyde may be vaporized. This process is in sharp contrast to conventional distillation techniques which must establish a constant boiling solution to volatize the product aldehyde, thereby increasing the time the catalyst solution must be heated, and consequently decomposing the rhodium bisphosphite complex.

Additionally, the thin film of mixture utilized by the falling film or short path distillation apparatus allows the mixture to be exposed to a greater vacuum during evaporation. This ability arises because the thin film does not suddenly form bubbles of low boiling point gases that can form from rapidly heating a solution during conventional distillation procedures which can cause the mixture to splatter and contaminate the distillate with precious catalyst which is then lost during downstream processing steps. As a result of the greater vacuum which can be achieved by the present invention, the boiling point of the product aldehyde, which is a function of pressure, can be reduced to a lower temperature than can be achieved with conventional distillation techniques. Therefore, the product/catalyst separation step of a hydroformylation process which employs a vacuum evaporation apparatus can be performed at lower temperature and therefore minimize catalyst decomposition.

Illustrative rhodium-bisphosphite complex catalyzed continuous hydroformylation processes in which such catalyst deactivation may occur include hydroformylation processes such as described, e.g., in U.S. Pat. Nos. 4,668,651; 4,774,361; 4,769,498; and 5,288,918 wherein the bisphosphite ligand is a ligand selected from the class consisting of Formulas (I)-(III) above, the entire disclosures of said patents being incorporated herein by reference thereto. Thus such hydroformylation processes and the conditions thereof are well known and it is to be understood that the particular manner in which the hydroformylation reaction is carried out and particular hydroformylation reaction conditions employed may be varied widely and tailored to meet individual needs and produce the particular aldehyde product desired.

In general, such hydroformylation reactions involve the production of aldehydes by reacting an olefinic compound with carbon monoxide and hydrogen in the presence of a rhodium-bisphosphite complex catalyst in a liquid medium that may also contains a solvent for the catalyst. The process may be carried out in a continuous single pass mode or more preferably in a continuous liquid catalyst recycle manner. The recycle procedure generally involves withdrawing a portion of the liquid reaction medium containing the catalyst and aldehyde product from the hydroformylation reaction zone, either continuously or intermittently, and distilling the aldehyde product therefrom in one or more stages, in a separate distillation zone in order to recover the aldehyde product and other volatile materials in vaporous form, the non-volatilized rhodium catalyst containing residue being recycled to the reaction zone. Likewise, the recovered non-volatilized rhodium catalyst containing residue can be recycled with or without further treatment to the hydroformylation zone in any conventional manner desired. Accordingly, the processing techniques of this invention may correspond to any known processing techniques such as heretofore employed in conventional liquid catalyst recycle hydroformylation reactions.

As noted above the hydroformylation reaction conditions that may be employed in the hydroformylation processes encompassed by this invention may include any suitable continuous hydroformylation conditions heretofore disclosed in the above-mentioned patents. For instance, the total gas pressure of hydrogen, carbon monoxide and olefinic unsaturated starting compound of the hydroformylation process may range from about 1 to about 10,000 psia. In general, however, it is preferred that the process be operated at a total gas pressure of hydrogen, carbon monoxide and olefinic unsaturated starting compound of less than about 1500 psia and more preferably less than about 500 psia. The minimum total pressure being limited predominately by the amount of reactants necessary to obtain a desired rate of reaction. More specifically the carbon monoxide partial pressure of the hydroformylation process of this invention is preferable from about 1 to about 120 psia, and more preferably from about 3 to about 90 psia, while the hydrogen partial pressure is preferably about 15 to about 160 psia and more preferably from about 30 to about 100 psia. In general H₂:CO molar ratio of gaseous hydrogen to carbon monoxide may range from about 1:10 to 100:1 or higher, the more preferred hydrogen to carbon monoxide molar ratio being from about 1:10 to about 10:1. Further, the hydroformylation process may be conducted at a reaction temperature from about 45° C to about 150°C. In general hydroformylation reaction temperature of about 50°C to about 120°C are preferred for all types of olefinic starting materials, the more preferred reaction temperatures being from about 50° C to about 100° C and most preferably about 95° C.

The olefinic starting material reactants that may be employed in the hydroformylation reactions encompassed by this invention include olefinic compounds containing from 6 to 30 carbon atoms. Such olefinic compounds can be terminally or internally unsaturated and be of straight-chain, branched chain or cyclic structures, as well as be olefin mixtures, such as obtained from the oligomerization of propene, butene, isobutene, etc., (such as so called dimeric, trimeric or tetrameric propylene, and the like, as disclosed, e.g., in U.S. Pat. Nos. 4,518,809 and 4,528,403). Moreover, such olefinic compounds may further contain one or more ethylenic unsaturated groups, and of course, mixtures of two or more different olefinic compounds may be employed as the starting hydroformylation material if desired. Further such olefinic compounds and the corresponding aldehyde products derived therefrom may also contain one or more groups or substituents which do not unduly adversely affect the hydroformylation process or the process of this invention such as described, e.g., in U.S. Pat. Nos. 3,527,809; 4,668,651 and the like.

Illustrative olefinic unsaturated compounds are alpha-olefins, internal olefins, alkyl alkenoates such as methyl-2-pentenoate, methyl-3-pentenoate and methyl-4-pentenoate, alkenyl alkanoates, alkenyl alkyl ethers, alkenols, and the like, e.g., 1-hexene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene, 2-hexene, 3-hexane, 2-heptene, cyclohexene, propylene dimers, propylene trimers, propylene tetramers, 2-ethyl-1-hexene, 2-octene, styrene, 3-phenyl-1-propene, 1,4-hexadiene, 1,7-octadiene, 3-cyclohexyl-1-butene, allyl alcohol, allyl butyrate, hex-1-en-4-ol, oct-1-en-4-ol, vinyl acetate, allyl acetate, 3-butenyl acetate, vinyl propionate, allyl propionate, methyl methacrylate, vinyl ethyl ether, vinyl methyl ether, allyl ethyl ether, n-propyl-7-octenoate, 3-butenenitrile, 5-hexenamide, 4-methyl styrene, 4-isopropyl styrene, 4-tert-butyl styrene, alpha-methyl styrene, 4-tert-butyl-alpha-methyl styrene, 1,3-diisopropenylbenzene, eugenol, iso-eugenol, safrole, iso-safrole, anethol, 4-allylanisole, indene, limonene, betapinene, dicyclopentadiene, cyclooctadiene, camphene, linalool, and the like.

Of course, it is understood that mixtures of different olefinic starting materials can be employed, if desired, by the hydroformylation process of the subject invention. More preferably the subject invention is especially useful for the production of aldehydes, by hydroformylating alpha olefins containing from 6 to 20 carbon atoms, including isobutylene, and internal olefins containing from 6 to 20 carbon atoms as well as starting material mixtures of such alpha olefins and internal olefins. Still more preferably the subject invention is especially useful for the production of aldehydes from methyl-pentenoates, in any isomeric form, or mixture thereof. These include methyl-4-pentenonate, trans-methyl-3-pentenonate, cis-methyl-3-pentenonate, trans-methyl-2-pentenonate and cis-methyl-2-pentenonate. It is also to be understood that commercial alpha olefins containing 6 or more carbon atoms may contain minor amounts of corresponding internal olefins and/or their corresponding saturated hydrocarbon and that such commercial olefins need not necessarily be purified from same prior to being hydroformylated.

As noted above, the continuous hydroformylation process of this invention involved the use of a rhodium-bisphosphite ligand complex catalyst as described herein. Of course mixtures of such catalysts can also be employed if desired. The amount of rhodium-phosphite complex catalyst present in the reaction medium of a given hydroformylation process encompassed by this invention need only be that minimum amount necessary to provide the given rhodium concentration desired to be employed and which will furnish the basis for at least the catalytic amount of rhodium necessary to catalyze the particular hydroformylation process involved such as disclosed e.g. in the above-mentioned patents. In general, rhodium concentrations in the range of from about 10 ppm to about 1000 ppm, calculated as free rhodium, in the hydroformylation reaction medium should be sufficient for most processes, while it is generally preferred to employ from about 10 to 500 ppm of rhodium and more preferably from 25 to 350 ppm to rhodium.

In addition to the rhodium-bisphosphite ligand complex catalyst the hydroformylation process encompassed by this invention may be carried out in the presence of free bisphosphite ligand, i.e. ligand that is not complexed with the rhodium metal of the complex catalyst employed. Said free bisphosphite ligand may correspond to any of the above defined bisphosphite ligands discussed above as employable herein. When employed it is preferred that the free bisphosphite ligand be the same as the bisphosphite ligand of the rhodium-bisphosphite complex catalyst employed. However, such ligands need not be the same in any given process. Moreover, while it may not be absolutely necessary for the hydroformylation process to be carried out in the presence of any such free bisphosphite ligand, the presence of at least some amount of free bisphosphite ligand in the hydroformylation reaction medium is preferred. Thus the hydroformylation process of this invention may be carried out in the absence or presence of any amount of free bisphosphite ligand, e.g. up to 100 moles, or higher per mole of rhodium metal in the hydroformylation reaction medium. Preferably the hydroformylation process of this invention is carried out in the presence of from about 1 to about 50 moles of bisphosphite ligand, and more preferably from about 1 to about 4 moles of bisphosphite ligand, per mole of rhodium metal present in the reaction medium; said amounts of bisphosphite ligand being the sum of both the amount of bisphosphite ligand that is bound (complexed) to the rhodium metal present and the amount of free (non-complexed) bisphosphite ligand present. Of course, if desired, make-up or additional bisphosphite ligand can be supplied to the reaction medium of the hydroformylation process at any time and in any suitable manner, e.g. to maintain a predetermined level of free ligand in the reaction medium.

The hydroformylation reactions encompassed by this invention may also be conducted in the presence of an organic solvent for the rhodium-bisphosphite complex catalyst and any free bisphosphite ligand that might be present. Any suitable solvent which does not unduly adversely interfere with the intended hydroformylation process can be employed. Illustrative suitable solvents for rhodium catalyzed hydroformylation processes include those disclosed e.g. in U.S. Pat. No. 4,668,651. Of course mixtures of one or more different solvents may be employed if desired. Most preferably the solvent will be one in which the olefinic starting material, catalyst, and weakly acidic additive if employed, are all substantially soluble. In general, it is preferred to employ aldehyde compounds corresponding to the aldehyde products desired to be produced and/or higher boiling aldehyde liquid condensation by-products as the primary solvent, such as the higher boiling aldehyde liquid condensation by-products that are produced in situ during the hydroformylation process. Indeed, while one may employ any suitable solvent at the start up of a continuous process, the primary solvent will normally eventually comprise both aldehyde products and higher boiling aldehyde liquid condensation by-products due to the nature of such continuous processes. Such aldehyde condensation by-products can also be performed if desired and used accordingly. Of course, the amount of solvent employed is not critical to the subject invention and need only be that amount sufficient to provide the reaction medium with the particular rhodium concentration desired for a given process. In general, the amount of solvent may range from 0 percent by weight up to about 95 percent by weight or more based on the total weight of the reaction medium.

Illustrative rhodium-bisphosphite complex catalysts employable in such hydroformylation reactions encompassed by this invention may include those disclosed in the above mentioned patents wherein the bisphosphite ligand is a ligand selected from the class consisting of Formulas (I), (II) and (III) above. In general, such catalysts may be preformed, or formed in situ, as described e.g., in said U.S. Pat. Nos. 4,668,651 and 4,769,498, and consist essentially of rhodium in complex combination with the organobisphosphite ligand. It is believed that carbon monoxide is also present and complexed with the rhodium in the active species. The active catalyst species may also contain hydrogen directly bonded to the rhodium.

As noted above illustrative organobisphosphite ligands that may be employed as the bisphosphite ligand complexed to the rhodium catalyst and/or any free bisphosphite ligand (i.e. ligand that is not complexed with the rhodium metal in the active complex catalyst) in such hydroformylation reactions encompassed by this invention include those of Formulas (I), (II), and (III) above.

Illustrative divalent radicals represented by R¹ in the above bisphosphite formulas (I), (II) and (III) include substituted and unsubstituted radicals selected from the group consisting of alkylene, alkylene-(Q)ₙ-alkylene, phenylene, naphthylene, phenylene-(Q)ₙ-phenylene and naphthylene-(Q)ₙ-naphthylene radicals, and where Q, and n are the same as defined above. More specific illustrative divalent radicals represented by R¹ are shown by the structure of (IV) or (V) wherein (Q)ₙ is the same as above. These include, 1,1'biphenyl-2,2'-diyl, 3,3'-dialkyl-1,1'-biphenyl-2,2'-diyl, 3,3'-dicarboxy ester-1,1'-biphenyl-2,2'-diyl, 1,1'binaphthyl-2,2'-diyl, 3,3'-dicarboxy ester-1,1'-binaphthyl-2,2'-diyl, 3,3'-dialkyl-1,1'-binaphthyl-2,2'-diyl, 2,2'-binaphthyl- 1,1'-diyl, phenylene-CH₂-phenylene, phenylene-O-phenylene, phenylene-CH(CH₃)-phenylene radicals, and the like.

Illustrative radicals represented by Z¹, Z², Z³, Z⁴, Z⁵, Z⁶, Z⁷, Z⁸, Z⁹, Z¹⁰, and Z¹¹ in above Formulas (IV) to (IX), in addition to hydrogen, include any of those organic substituents containing from 1 to 18 carbon atoms, disclosed in U.S. Pat. No. 4,668,651, or any other radical that does not unduly adversely effect the process of this invention. Illustrative radicals and substituents encompass alkyl radicals, including primary, secondary and tertiary alkyl radicals such as methyl, ethyl n-propyl, isopropyl, butyl, sec-butyl, t-butyl, neo-pentyl, n-hexyl, amyl, sec-amyl, t-amyl, iso-octyl, decyl, octadecyl, and the like; aryl radicals such as phenyl, naphthyl and the like; aralkyl radicals such as benzyl, phenylethyl, triphenylmethyl, and the like; alkaryl radicals such as tolyl, xylyl, and the like; condensated aryl radicals such as phenylene, naphthylene, and the like, alicyclic radicals such as cyclopentyl, cyclohexyl, 1-methylcyclohexyl, cyclooctyl, cyclohexylethyl, and the like; alkoxy radicals such as methoxy, ethoxy, propoxy, t-butoxy― OCH₂CH₂OCH₃, ―O(CH₂CH₂)₂OCH₃, ―O(CH₂CH₂)₃OCH₃, and the like; aryloxy radicals such as phenoxy and the like; as well as silyl radicals such as ―Si(CH₃)₃, ―Si(OCH₃)₃, ―Si(C₃H₇)₃, and the like; amino radicals such as ― NH₂, ―N(CH₃) ₂, ―NHCH₃, ―NH(C₂H₅), and the like; acyl radicals such as ― C(O)CH₃, ―C(O)C₂H₅, ―C(O)C₆H₅, and the like; carbonyloxy radicals such as ―C(O)OCH₃, ―C(O)OCH(CH₃)₂ ―(C(O)CH(CH₃)C₈H₁₇, and the like; oxycarbonyl radicals such as ―O(CO)C₆H₅, and the like; amido radicals such as ―CONH₂, ―CON(CH₃)₂, ―NHC(O)CH₃, and the like; sulfonyl radicals such as ―S(O)₂C₂H₅ and the like; sulfinyl radicals such as ―S(O)CH₃ and the like; thionyl radicals such as ―SCH₃, ―SC₂H₅, ―SC₆H₅, and the like; phosphonyl radicals such as ―P(O)(C₆H₅)₂, ―P(O)(CH₃)₂, ―P(O)(C₂H₅)₂, ―P(O)(C₃H₇)₂, P(O)CH₃(C₆H₅), ―P(O)(H)(C₆H₅), and the like.

Illustrative radicals represented by X¹, X², X³, X⁴, X⁵ and X⁶ in above Formulas (IV) to (IX) include those illustrated and discussed above as representing Z¹ to Z¹¹, except hydrogen and condensated aryl radicals.

Illustrative radicals represented by Y¹, Y², Y³, Y⁴ and Y⁵ in above Formulas (IV) to (IX) include those illustrated and discussed above as representing Z¹ to Z¹¹, except condensated aryl radicals.

More preferably, X¹ is the same as X² and Z¹ is the same as Z² in Formula (IV), X³ is the same as X⁴, Z³ is the same as Z⁴, and Y¹, Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).

Specific illustrative examples of the bisphosphite ligands employable in this invention include such preferred ligands as:
3,3'-bis(carboxyisopropyl)-1,1'-binaphthyl-2,2'-diyl-bis[bis(1-naphthyl)]phosphite having the formula: 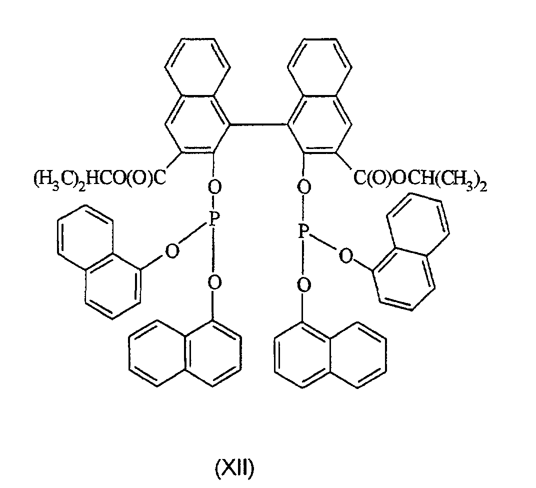
[3,3'-bis(t-butyl)-5,5'-dimethoxy-1,1'-biphenyl-2,2'-diyl]-bis(oxy)]-bis(dibenzo[d,f] [1,3,2])dioxaphosphepin having the formula: 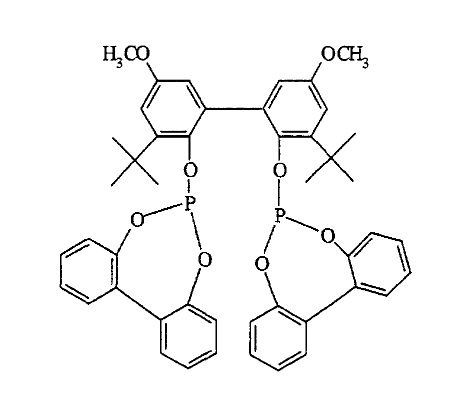
3,3'-bis(carboxyisopropyl)-1,1'-binaphthyl-2-yl-bis[(1-naphthyl)]phosphite-2'-yl-oxy-dibenzo[d,f] [1,3,2]dioxaphosphepin having the formula: 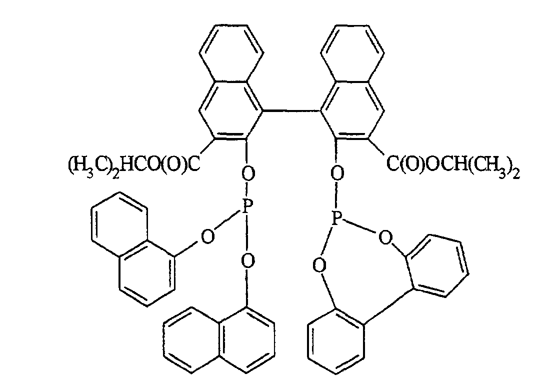
5,5'-bis(t-butyl)-3,3'-dimethoxy-1,1'-biphenyl-2,2'-diyl-bis[bis(1-naphthyl)]phosphite having the formula: 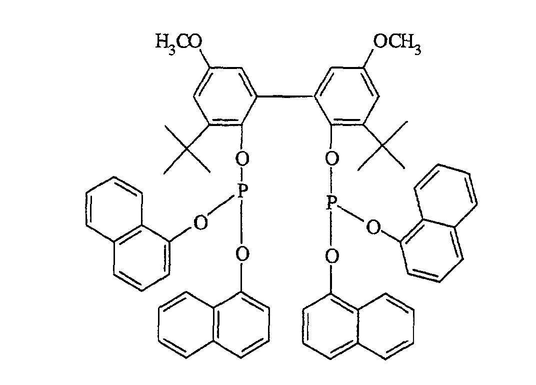
3,3'-bis(carboxymethyl)-1,1'-binaphthyl-2,2'-diyl-bis[bis(2-t-butylphenyl)]phosphite having the formula: 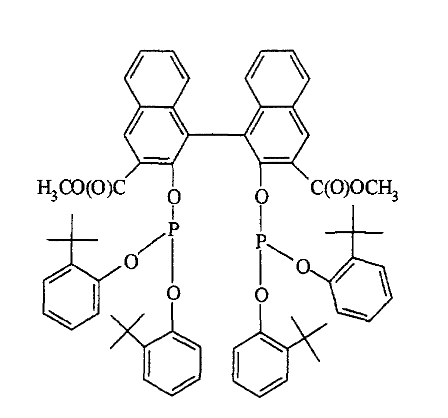
[3,3'-bis(t-butyl)-5,5'-dimethoxy-1,1'-biphenyl-2,2'-diyl]-bis(oxy)]-bis([1,1'-dinaphto[d,f] [1,3,2])dioxaphosphepin having the formula: 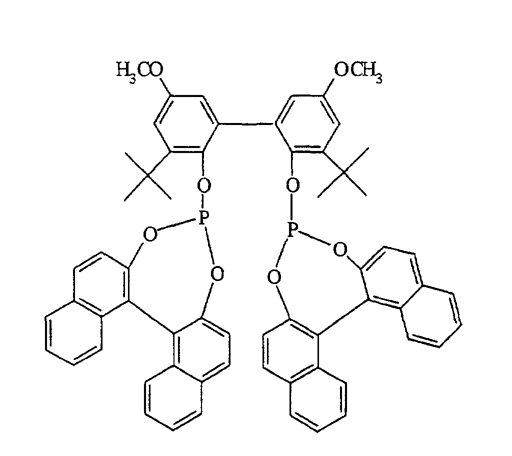
and the like.

Such types of bisphosphite ligands employable in this invention and/or methods for their preparation are well known as seen disclosed for example in U.S. Pat. Nos. 4,668,651; 5,288,918; 5,710,306, the entire disclosure of which is incorporated herein by reference thereto.

Of course it is to be understood that while the optimization of the subject invention necessary to achieve the best results and efficiency desired are dependent upon one's experience in the utilization of the subject invention, only a certain measure of experimentation should be necessary to ascertain those conditions which are optimum for a given situation and such should be well within the knowledge of one skilled in the art and easily obtainable by following the more preferred aspects of this invention as explained herein and/or by simple routine experimentation.

Finally, the aldehyde products of the hydroformylation process of this invention have a wide range of utility that is well known and documented in the prior art e.g. they are especially useful as starting materials for the production of alcohols and acids, as well as for the production of useful monomeric and polymeric compounds such as ε-caprolactam, nylon-6, adipic acid and nylon-6,6.

The following examples are illustrative of the present invention and are not be regarded as limitative. It is to be understood that all of the parts, percentages and proportions referred to herein and in the appended claims are by weight unless otherwise indicated.

### EXAMPLE 1

This example demonstrates that the undesired ligand consumption rate is improved by operating the catalyst-product separator at low temperature and residence time. This low temperature is achieved through low operating pressure in a vacuum wiped falling film evaporator.

In a continuous process according to figure II the substrate methyl-3-pentenoate is reacted with CO and H2 to form a mixture of methyl formyl valerates. This is done by contacting methyl-3-pentenoate with a catalyst comprising Rh, 3,3'-bis(carboxyisopropyl)-1,1'-binaphthyl-2,2'-diyl-bis[bis(1-naphthyl)]phosphite ligand in slight molar excess to rhodium, and a mixture of CO and H2 (reaction conditions were as follows: 95°C, CO:H2 = 1:1 mole:mole, P = 5 bara, mean residence time = 6 hours total in three well mixed reactors in series). The reactor effluent is flashed to 1 bar to remove most of the dissolved gases which are subsequently purged. After cooling to less than 40 °C, the liquid is passed over a dry, weakly basic ion exchange resin to remove acidic ligand degradation products. The catalyst concentration and free ligand concentration in the effluent is monitored regularly by on-line HPLC.

By feeding fresh ligand solution to the reactor section, a constant molar ratio of Rh:ligand of 1:1.2 mole:mole is maintained in the reactor effluent. When the rate of ligand supply is equal to the degradation rate, the active ligand concentration stays constant and the process is operated in steady state. Below it is shown that this steady state degradation rate is dependent on the temperature in the catalyst product separation.

After flashing and passing the ion exchange resin, the reactor effluent is fed to a wiped falling film evaporator. The evaporation rate is controlled by the temperature of the heating oil used as the heating utility for this evaporator. The heating oil temperature is controlled such that 75 wt% of the feed to the evaporator is going overhead to the product work-up section. The remaining 25 wt% contains the catalyst and is recycled as the evaporator bottoms to the reactor. The mean residence time of the catalyst in the separation unit is less than 10 minutes, of which less than 2 minutes are spent on the heating surface, the most critical area for the catalyst. Figure I shows the ligand consumption in mg ligand/kg product as a function of the thermal load. Clearly, the lower the thermal load, the lower the ligand degradation rate and the more economic the process.

At an operating pressure of 40 mbar and an oil temperature of 160°C, the ligand is quantitatively degraded during the catalyst/product separation step which corresponds to ligand consumption > 5000 mg/kg product.
The following describe the lettered and numbered points in Figure II:
1. Ligand make-up stream
2. Other process feeds (e.g., CO, H₂, methyl-3-pentenoate)
3. Purge gases
4. Distillate to product work-up
5. Catalyst recycle stream
6. Heating oil

A. Reactor section
B. Flash section
C. Ion exchange section
D. Hot oil heated wiped film evaporator.

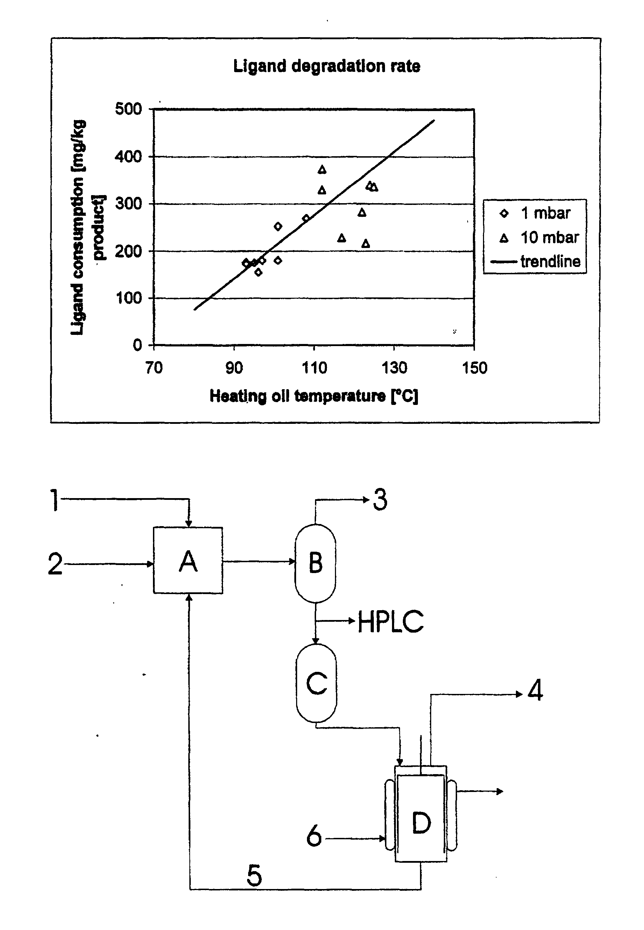

## Claims

1. A continuous hydroformylation process for forming an aldehyde comprising:
A) reacting an olefinically unsaturated compound containing from 6 to 30 carbon atoms, carbon monoxide, hydrogen, and a rhodium-bisphosphite ligand complex catalyst to form a reaction mixture
B) evaporating at least a portion of said liquid reaction mixture to isolate aldehyde product
C) recycling the catalyst after the evaporation,
wherein said evaporation is accomplished in a vacuum evaporator with a short mean residence time and a moderate thermal exposure of the liquid reaction mixture in the evaporator.

2. The process of claim 1, wherein the heating surface of the evaporator is maintained at a temperature below 125 °C.

3. The process of claim 2, wherein the heating surface of the evaporator is maintained at a temperature below 110 °C.

4. The process of any one of claims 1-3,wherein said evaporation is performed at a pressure below 200 mbar

5. The process of claim 4, wherein said evaporation is performed at a pressure below 50 mbar.

6. The process of claim 5, wherein said evaporation is performed at a pressure below 20 mbar.

7. The process of claim 6, wherein said evaporation is performed at a pressure below 10 mbar.

8. The process according to any one of claims 1-7, wherein the residence time of the liquid reaction mixture in the evaporator is less than 15 minutes.

9. The process according to claim 8, wherein the residence time is less than 10 minutes.

10. The process according to claim 9,wherein the residence time is less than 5 minutes.

11. The process according to any one of claims 1-10, wherein the vacuum evaporator is a short path evaporator.

12. The process according to any one of claims 1-11, wherein the vacuum evaporator is a falling film evaporator.

13. The process according to any one of claims 1-12, wherein the vacuum evaporator is a wiped falling film evaporator.

14. The process of any one of claims 1-13, wherein the bisphosphite ligand of said complex catalyst is a ligand of a formula selected from the group consisting of: 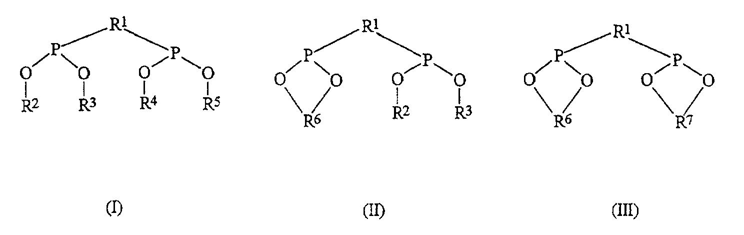 wherein each R¹ represents a divalent radical selected from a group consisting of alkylene, alkylene-(Q)ₙ-alkylene, arylene and arylene-(Q)ₙ-arylene, and wherein each alkylene radical individually contains from 2 to 18 carbon atoms and is the same or different, and wherein each arylene radical individually contains from 6 to 18 carbon atoms and is the same or different; wherein each Q individually represents a divalent bridging group of ―O―or ―CR'R"― wherein each R' and R" radical individually represents hydrogen or a methyl radical; and wherein each n individually has a value of 0 or 1,
wherein R², R³, R⁴, and R⁵ might be the same or different and each is individually represented by the structure of (VI) or (VII), 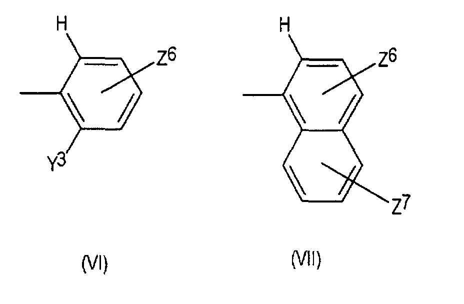 wherein R⁶ and R⁷ might be the same or different and each is individually represented by the structure of (VIII) or (IX), 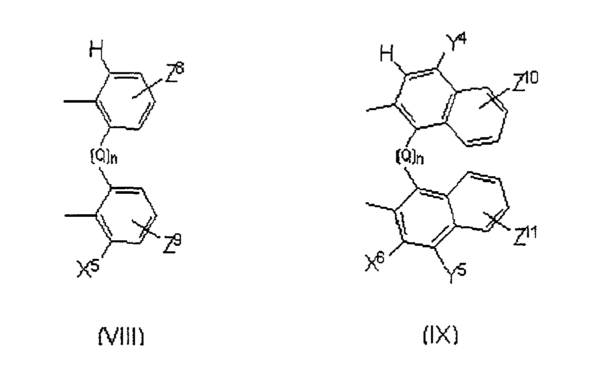 wherein X¹, X², X³, X⁴, X⁵ and X⁶ might be the same or different and each individually represents an organic radical, wherein Y¹, Y², Y³, Y⁴ and Y⁵ are the same or different and each represents a hydrogen or alkyl radical,
wherein Z¹, Z², Z³, Z⁴, Z⁵, Z⁶, Z⁷, Z⁸, Z⁹, Z¹⁰ and Z¹¹ might be the same or different and each represent a hydrogen or an organic radical placed at any remaining position of the aryl rings of structures (IV) to (V),

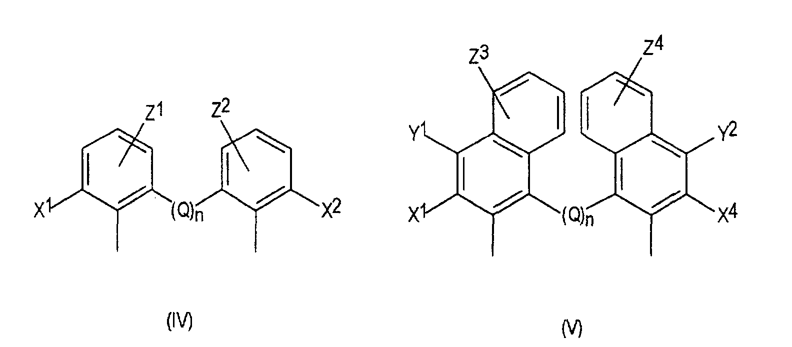

15. The process of claim 14, wherein R¹ is represented by the structure of (IV), (V), (VII), (VIII) or (IX)wherein X¹ is the same as X² and Z¹ is the same as Z² in Formula (IV), X³ is the same as X⁴, Z³ is the same as Z⁴, and Y¹ and Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).

16. The process according to any one of claims 14-15, wherein the ligand used is [3,3'-bis(t-butyl)-5,5'-dimethoxy-1,1 '-biphenyl-2,2'-diyl]-bis(oxy)]-bis(dibenzo[d,f][1,3,2])dioxaphosphepin, 3,3'-bis(carboxyisopropyl)-1,1'-binaphthyl-2,2-diyl-bis[bis(1-naphthyl)]phosphite and 3,3'-bis(carboxymethyl)-1,1'-binaphthyl-2,2'-diyl-bis[bis(2,5-di-t-butyl)]phosphite.
